# EUROPEAN PATENT SPECIFICATION

(11) **EP 0 989 222 B1**
(45) Date of publication and mention of the grant of the patent: **27.06.2007**
(21) Application number: 99909287.7
(22) Date of filing: 23.03.1999
(51) Int. Cl.: D04H 3/16, D04H 3/14, B32B 5/26, B32B 27/32, B32B 5/02, A61F 13/15, D04H 13/00, D01F 8/06

(54) **FLEXIBLE NONWOVEN FABRIC LAMINATE**
FLEXIBLES LAMINAT AUS FASERVLIES
STRATIFIE DE NON-TISSE FLEXIBLE

(30) Priority: 24.03.1998 JP 7545498
(43) Date of publication of application: 29.03.2000
(73) Proprietor: Mitsui Chemicals, Inc., Tokyo (JP)
(72) Inventor: HISADA, Minoru, Yokkaichi-shi Mie 512-8054 (JP); ISHII, Hiroshi, Yokkaichi-shi Mie 512-8054 (JP); MOTOMURA, Shigeyuki, Yokkaichi-shi Mie 512-8054 (JP); TAKESUE, Kunihiko, Yokkaichi-shi Mie 512-8054 (JP)
(74) Representative: HOFFMANN EITLE
(86) International application number: PCT/JP1999/001426
(87) International publication number: WO 1999/049120

(56) References cited:
- EP-A- 0 395 336
- EP-A- 0 625 221
- EP-A- 0 691 427
- EP-A- 0 959 165
- EP-A1- 0 546 837
- WO-A-97/34037
- DE-A- 3 734 963
- JP-A- 5 230 754
- JP-A- 9 273 060

## Description

### TECHNICAL FIELD

The present invention relates to a soft nonwoven fabric laminate. Specifically, it relates to a soft nonwoven fabric laminate having satisfactory uniformity, excellent softness, gas permeability and water impermeability which is fit for use as a material for sanitary goods such as disposable diapers and a packaging material.

### TECHNICAL BACKGROUND

In recent years, nonwoven fabrics are used for a wide variety of applications because of their excellent gas permeability and solftness and are finding an increasing number of applications. Against this background, nonwoven fabrics are being called on to have varied properties meeting the requirements of individual applications and to have improved properties.

Document WO-A-9 734 037 discloses nonwoven fabrics laminates comprising at least one layer of meltblown elastic fibers bonded on either side with a layer of soft non-elastic fibers; they are used in garments, personal case products and are soft and breathable.

In the meantime, spunbonded nonwoven fabrics have been used as the surface materials of absorptive goods such as disposable diapers because they do not have napping and yet show excellent resistance to loose fibers. However, spunbonded nonwoven fabrics do not generally have satisfactory uniformity (showing a variation in thickness) and therefore are called on to have improved uniformity. As a method for improving the uniformity of spunbonded nonwoven fabrics, the method in which a meltblown nonwoven fabric is laminated to a spunbonded nonwoven fabric is known. However, such laminate is generally inferior in softness and does not show adequate adhesion between the spunbonded nonwoven fabric layer and the meltblown nonwoven fabric layer in some cases.

Therefore, the present invention was made against the background as described above and is intended to provide a soft nonwoven fabric laminate having satisfactory uniformity and excellent softness, gas permeability and water impermeability and showing excellent interlaminar bonding strength.

### DISCLOSURE OF THE INVENTION

The inventors of the present invention made a study in the circumstance as described above and consequently have come to complete the present invention after finding that the use of a spunbonded nonwoven fabric comprising a particular conjugate fiber as the spunbonded nonwoven fabric and a meltblown nonwoven fabric comprising a polyolefin composition comprising a particular polyolefin-based elastomer and a propylene-based polymer as the meltblown nonwoven fabric makes it possible to obtain a laminate having satisfactory uniformity and excellent softness, gas permeability and water impermeability and showing excellent interlaminar bonding strength (peel strength).

The soft nonwoven fabric laminate is a laminate which comprises at least one spunbonded nonwoven fabric layer and at least one meltblown nonwoven fabric layer and one surface or both surfaces of which are a spunbonded nonwoven fabric layer; with the aforesaid spunbonded nonwoven fabric comprising a conjugate fiber:
which comprises:
   (A) A propylene-based polymer (a) having the physical properties as described in (1) and (2) below:
      (1) The ethylene content is 0 to 5 mol%;
      (2) Mw/Mn is 2 to 4;
   (B) An ethylene-based polymer (b) having the physical properties as described in (3) and (4) below and forming at least part of the fiber surface:
      (3) The density is 0.880 to 0.970 g/cm³;
      (4) Mw/Mn is 1.5 to 4; and
   in which the ratio by weight between (a) and (b) ((a)/(b)) is 5/95 to 50/50; and
with the aforesaid meltblown nonwoven fabric comprising a polyolefin composition which comprises a polyolefin-based elastomer (c) and a propylene-based polymer (d) and contains the polyolefin-based elastomer (c) at a ratio of not less than one wt% but not more than 50 wt%.

Good examples of the conjugate fibers forming the aforesaid spunbonded nonwoven fabric include (1) a concentric core-sheath-type conjugate fiber made up of the core comprising the propylene-based polymer (a) and the sheath comprising the ethylene-based polymer (b), (2) an eccentric core-sheath-type conjugate fiber made up of the core comprising the propylene-based polymer (a) and the sheath comprising the ethylene-based polymer (b), and (3) a side-by-side-type (bimetal type) conjugate fiber made up of the propylene-based polymer (a) and the ethylene-based polymer (b).

In the present invention, the aforesaid spunbonded nonwoven fabric layer and the aforesaid meltblown nonwoven fabric layer are preferably bonded by heat embossing processing. In this case, the bonded area of the spunbonded nonwoven fabric layer and the meltblown nonwoven fabric layer is preferably 5% to 35% of the area of contact between the spunbonded nonwoven fabric layer and the meltblown nonwoven fabric layer.

The soft nonwoven fabric laminate of the present invention has preferably the KOSHI value of less than 10, and the peel strength between the spunbonded nonwoven fabric layer and the meltblown nonwoven fabric layer is preferably not less than 30 g/5 cm.

Further, the present invention provides a soft nonwoven fabric laminate suitable especially for use in materials for sanitary goods and packaging materials.

### BRIEF EXPLANATION OF THE DRAWINGS

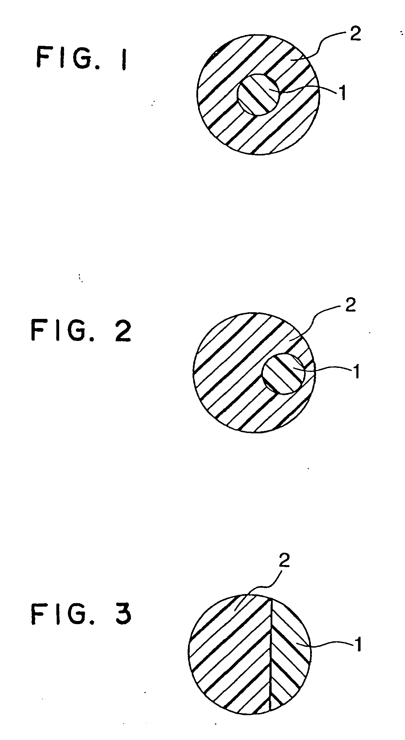

Fig. 1 shows a representative sectional view of the concentric core-sheath-type conjugate fiber. Fig. 2 shows a representative sectional view of the eccentric core-sheath-type conjugate fiber, and Fig. 3 shows a representative sectional view of the side-by-side-type conjugate fiber.

### BEST EMBODIMENT OF THE INVENTION

The soft nonwoven fabric laminate of the present invention is a laminate which comprises at least one spunbonded nonwoven fabric layer and at least one meltblown nonwoven fabric layer and one surface or both surfaces of which are a spunbonded nonwoven fabric layer.

The spunbonded nonwoven fabric comprising the soft nonwoven fabric laminate of the present invention is made up of a conjugate fiber which comprises the propylene-based polymer (a) and the ethylene-based polymer (b), in which the ratio by weight between (a) and (b) ((a)/(b)) is in the range of 5/95 to 50/50, preferably 10/90 to 40/60, more preferably 10/90 to 20/80, and at least part of the surface of the fiber of which is made up of (b). If the weight ratio of the propylene-based polymer (a) to 100 by weight of the conjugate fiber is less than 5 , the strength of the conjugate fiber may be inadequate in some cases; and if the weight ratio of the propylene-based polymer (a) to 100 by weight of the conjugate fiber is more than 50, the conjugate fiber may show poor softness in some cases.

Preferable examples of the aforesaid conjugate fibers are: (1) a concentric core-sheath-type conjugate fiber made up of the core comprising the propylene-based polymer (a) and the sheath comprising the ethylene-based polymer (b), (2) an eccentric core-sheath-type conjugate fiber made up of the core comprising the propylene-based polymer (a) and the sheath comprising the ethylene-based polymer (b), and (3) a side-by-side-type conjugate fiber made up of the propylene-based polymer (a) and the ethylene-based polymer (b). Further, out of these conjugate fibers, the eccentric core-sheath-type conjugate fiber of (2) above and the side-by-side-type conjugate fiber of (3) above become crimped fibers and are more preferable with respect to softness.

Fig. 1 shows a representative sectional view of a conjugate fiber. Fig. 1 shows a representative sectional view of the concentric core-sheath-type conjugate fiber. Fig. 2 shows a representative sectional view of the eccentric core-sheath-type conjugate fiber, and Fig. 3 shows a representative sectional view of the side-by-side-type conjugate fiber. In Figs. 1 through 3, 1 represents the part made up of the propylene-based polymer (a), 2 represents the part made up of the ethylene-based polymer (b).

The propylene-based polymer (a) forming the conjugate fiber is a propylene homopolymer or propylene-based polymer having the ethylene content of 0 to 5 mol%. The propylene-based polymer (a), preferably from the viewpoint of spinnability, has the melt flow rate (MFR, as determined at a load of 2.16 kg and at 230°C in accordance with ASTM D1238) in the range of preferably 20 to 100 g/10 minutes, more preferably 30 to 70 g/10 minutes. Furthermore, the Mw/Mn (Mw: weight-average molecular weight; Mn: number-average molecular weight) is preferably from the viewpoint of spinnability in the range of 2 to 4. The term preferable spinnability as herein used means the resin's property of being capable of being spun stably without involving the breakage of the filament when the melted resin is spun from the spinneret. Further, the Mw/Mn may be determined by the method already known to the public using the gel permeation chromatography.

Examples of the ethylene-based polymer (b) forming the conjugate fiber include homopolymer of ethylene (manufactured by either low-pressure or high-pressure process) and random copolymers of ethylene with α-olefins such as propylene, 1-butene, 1-hexene, 4-methyl-1-pentene and 1-octene. These ethylene-based polymers (b) has preferably from the viewpoint of spinnability density in the range of 0.880 to 0.970 g/cm³, preferably 0.900 to 0.950 g/cm³. Furthermore, their MFR (as determined at a load of 2.16 kg and at 190°C in accordance with ASTM D1238) is preferably from the viewpoint of spinnability in the range of preferably 20 to 60 g/10 minutes, and the Mw/Mn is preferably from the viewpoint of spinnability in the range of 1.5 to 4, preferably 2 to 4.

As the ethylene-based polymer (b), an ethylene homopolymer whose density, MFR and Mw/Mn are in the aforesaid ranges is preferable from the viewpoint of the solftness and spinnability of the nonwoven fabric obtained therefrom.

The nonwoven fabric comprising the aforesaid conjugate fiber shows better softness than the conventional nonwoven fabrics comprising polypropylene resin because most or all of the conjugate fiber surfaces forming the aforesaid nonwoven fabric is made up of the ethylene-based polymer (b). Furthermore, if the conjugate fiber forming the nonwoven fabric is crimped fiber, the nonwoven fabric shows much better softness.

In the present invention, a slip agent such as oleic amide, erucic amide and stearic amide may be added in a ratio of 0.1 to 0.5 wt% to the aforesaid ethylene-based polymer (b). The addition of a slip agent to the ethylene-based polymer improves the loose-fiber resistance of the resulting spunbonded nonwoven fabric. Moreover, in the present invention, a slip agent may be added to the propylene-based polymer (a).

Furthermore, in the present invention, other polymers, coloring agents, heat stabilizers, nucleating agents, etc. may be added to the propylene-based polymer (a) and/or the ethylene-based polymer (b) as required to the extent that the purpose of the present invention is not thwarted.

The method already known to the public may be used for the method for manufacturing the spunbonded nonwoven fabric comprising the conjugate fiber. Under such method, for example, the ratio by weight between the propylene-based polymer (a) and the ethylene-based polymer (b) is set at 5/95 to 50/50, conjugate fiber filaments are spun by the two-extruder melting and spinning method, the filaments thus spun are cooled with a cooling fluid, and then the filaments are given tension by means of stretching air so that the filaments become a desired fineness. Thereafter, the spun filaments are collected on a collection belt and subjected to the processing of tangling so that the spunbonded nonwoven fabric is obtained. Examples of the tangling processing method include, for example, a method in which the fibers are fused by the processing of heat embossing, a method in which the fibers are fused by ultrasonic heating, a method in which the fibers are entangled by using a water jet, a method in which the fibers are fused by passing hot air through them, and a method in which the fibers are entangled by using a needle punch. Out of these entangling methods, the process of heat embossing is preferable. The diameter of the fiber forming this spunbonded nonwoven fabric is normally approximately 5 to 30 µm (0.2 to 7 denier), preferably approximately 10 to 20 µm.

The fiber forming the meltblown nonwoven fabric used in the present invention comprises a polyolefin composition which is made up of the polyolefin-based elastomer (c) and the propylene-based polymer (d) and contains the polyolefin-based elastomer (c) at a ratio of not less than one wt% but not more than 50 wt%, preferably 10 to 40 wt%, more preferably 20 to 35 wt%. When the polyolefin-based elastomer (c) content is less than one wt%, the adhesion between the spunbonded nonwoven fabric layer and the meltblown nonwoven fabric layer becomes inadequate in some cases; when the rate of the polyolefin-based elastomer (c) content exceeds 50 wt%, the spinnability becomes poor in some cases.

The olefin-based elastomer (c) composing the aforesaid polyolefin composition is a polymer or copolymer of α-olefin having 2 to 20 carbon atoms, which has preferably the density in the range of not more than 0.900 g/cm³, more preferably 0.860 to 0.900 g/cm³, and the MFR (as determined at a load of 2.16 kg and at 190°C in accordance with ASTM D1238) in the range of 0.01 to 150 g/10 minutes, more preferably 20 to 100 g/10 minutes. Such olefin-based elastomer (c) preferably has the crystallinity of less than 30% as measured by X-ray diffractometry, or is amorphous.

Examples of the α-olefin having 2 to 20 carbon atoms include ethylene, propylene, 1-butene, 1-pentene, 1-hexane, 4-methyl-1-pentene, 1-octene, 1-decene and blends thereof. Out of these, α-olefins having 2 to 10 carbon atoms are preferably, and especially ethylene and 1-butene are preferable.

The olefin-based elastomer (c) may contain component units other than those component units derived from α-olefin, such as those component units derived from a diene compound, to the extent that will not impair the properties of the olefin-based elastomer (c). For example, examples of the component units that may be contained in the olefin-based elastomer (c) used in the present invention include those component units derived from chain nonconjugated dienes such as 1,4-hexadiene, 1,6-octadiene, 2-methyl-1,5-hexadiene, 6-methyl-1,5-heptadiene and 7-methyl-1,6-octadiene; cyclic nonconjugated dienes such as cyclohexadiene, dicyclopentadiene, methyltetrahydroindene, 5-vinylnorbornene, 5-ethylidene-2-norbornene, 5-methylene-2-norbornene, 5-isopropyridene-2-norbornene and 6-chloromethyl-5-isopropenyl-2-norbonene; and diene compounds such as 2,3-diisopropyridene-5-norbonene, 2-ethylidene-3-isopropyridene-5-norbornene and 2-propenyl-2,2-norbornadiene. These diene compounds may be used singly or in combination with one another. The content of such diene component is normally not more than 10 mol%, preferably not more than 5 mol%.

Specific examples of the olefin-based elastomer (c) include polymers and copolymers comprising 0 to 95 mol%, preferably 30 to 92 mol%, more preferably 50 to 90 mol% of the component unit derived from ethylene, 1 to 100 mol%, preferably 4 to 70 mol%, more preferably 8 to 50 mol% of the component unit derived from α-olefin having 3 to 20 carbon atoms and 0 to 10 mol%, preferably 0 to 5 mol%, more preferably 0 to 3 mol% of the component unit derived from diene compound. More specific examples include ethylene-α-olefin copolymers containing 10 to 50 mol% of α-olefins having 3 to 10 carbon atoms, such as propylene, 1-butene, 1-pentene, 1-hexene, 4-methyl-1-pentene, 1-octene and 1-decene and propylene-α-olefin copolymers containing 10 to 50 mol% of α-olefins having 4 to 10 carbon atoms, such as 1-butene, 1-pentene, 1-hexene, 4-methyl-1-pentene, 1-octene and 1-decene.

Examples of the propylene-based polymer (d) composing the aforesaid polyolefin composition include a propylene homopolymer or random copolymers of propylene and not more than 10 mol% of α-olefin, other than propylene, having 2 to 20, preferably 2 to 10, carbon atoms, such as ethylene, 1-butene, 1-hexene and 4-methyl-1-pentene. The propylene-based polymer (d) has preferably the MFR (as determined at a load of 2.16 kg and at 230°C in accordance with ASTM D1238) in the range of preferably 30 to 1,500 g/10 minutes, more preferably 400 to 1,000 g/10 minutes and the Mw/Mn in the range of preferably 2 to 4. As the propylene-based polymer (d), a propylene homopolymer is preferable.

The use of a meltblown nonwoven fabric formed from the fiber comprising the polyolefin composition as described above makes it possible to obtain a laminate with excellent bonding strength when the meltblown nonwoven fabric is bonded with a spunbonded nonwoven fabric by the processing of heat embossing.

Methods already known to the public may be used for the method for manufacturing the meltblown nonwoven fabric used in the present invention. For example, the meltblown nonwoven fabric may be manufactured by melting the aforesaid composition comprising the polyolefin elastomer (c) and the propylene-based polymer (d) by means of an extruder, extruding it through the meltblown fiber spinneret, blow-spinning the extruded fiber as a flow of fine fiber by means of a high-temperature/high-velocity fluid to cause the fine fiber to be deposited on the collection device form a fine fiber web, and subjecting it to the processing of heat fusion as required. The average diameter of the fiber forming this meltblown nonwoven fabric is normally approximately 1 to 30 µm, preferably approximately 2 to 10 µm.

The soft nonwoven fabric laminate of the present invention comprises at least one spunbonded nonwoven fabric layer and at least one meltblown nonwoven fabric layer. The layer structure is not restricted in any particular way so long as at least one of the surface layers is made up of a spunbonded nonwoven fabric layer, but a preferable layer structure is a spunbonded nonwoven fabric layer/a meltblown nonwoven fabric layer and a spunbonded nonwoven fabric layer/a meltblown nonwoven fabric layer/a spunbonded nonwoven fabric layer.

The basis weights of the spunbonded nonwoven fabric and meltblown nonwoven fabric of the soft nonwoven fabric laminate of the present invention may be selected as appropriate to meet the requirements of the particular applications of the soft nonwoven fabric laminate and the quality, economics, etc. required thereof. The basis weight of the laminate is normally approximately 10 to 50 g/m², more preferably approximately 12 to 30 g/m².

Any method may be used for the method for manufacturing the soft nonwoven fabric laminate of the present invention, and there is no particular limit to the method so long as it is capable of forming the laminate by laminating the spunbonded nonwoven fabric and the meltblown nonwoven fabric to form into one laminate. Examples of the aforesaid method for manufacturing the soft nonwoven fabric laminate include (1) a method in which a two-layer laminate is made by thermally bonding the spunbonded nonwoven fabric and the meltblown nonwoven fabric after the meltblown nonwoven fabric is formed by depositing the fiber formed by the meltblown method directly on the spunbonded nonwoven fabric, (2) a method in which a three-layer laminate is made by forming a meltblown nonwoven fabric by depositing the fiber formed by the meltblown method directly on the spunbonded nonwoven fabric <1>, forming the spunbonded nonwoven fabric <2> by depositing the fiber formed by the spunbonding method directly on the aforesaid meltblown nonwoven fabric, and then thermally bonding the spunbonded nonwoven fabric <1>, the meltblown nonwoven fabric and the spunbonded nonwoven fabric <2>, (3) a method in which a laminate is made by putting a spunbonded nonwoven fabric and a meltblown nonwoven fabric on top of each other and thermally bonding the two nonwoven fabrics by heating and pressing, and (4) a method in which a laminate is made by bonding a spunbonded nonwoven fabric and a meltblown nonwoven fabric by use of an adhesive such as hot melt adhesives and solvent-based adhesives.

The soft nonwoven laminate of the present invention has adequate adhesion even when the meltblown nonwoven fabric is bonded with a spunbonded nonwoven fabric comprising a conjugate fiber made up of a propylene-based polymer and an ethylene-based polymer by means of the thermal bonding method such as the processing of heat embossing. The reason is that the meltblown nonwoven fabric is made of a polyolefin composition comprising a particular polyolefin-based elastomer and a propylene-based polymer.

The method in which the meltblown nonwoven fabric is formed directly on the spunbonded nonwoven fabric may be accomplished by the meltblown method in which a melted material of an polyolefin composition is extruded into a flow of fine fiber from the meltblown spinneret and blown onto the surface of the spunbonded nonwoven fabric before the flow of fine fiber solidifies completely, so that fine fiber may be deposited on the surface of the spunbonded nonwoven fabric. At this time, while the reverse surface of the surface of the spunbonded nonwoven fabric onto which the fine fiber formed by the meltblown method is blown is placed under negative pressure, the fine fiber is blown and deposited onto the spunbonded nonwoven fabric placed on the collection surface of a mesh belt, for example, and at the same time, the spunbonded nonwoven fabric and the meltblown nonwoven fabric are combined into one so that a soft nonwoven fabric laminate having the spunbonded nonwoven fabric and the meltblown nonwoven fabric is obtained. If these two nonwoven fabrics are not combined into one in an inadequate manner, they may be combined into one adequately by means of heated embossing press rolls, for example.

In the event of producing a 3-layer nonwoven fabric laminate by the method (3) above, such laminate may be produced by depositing a conjugate filament spun by the conjugate melt-spinning method on the meltblown nonwoven fabric layer of the aforesaid soft nonwoven fabric laminate having the spunbonded nonwoven fabric layer and the meltblown nonwoven fabric layer and then thermally bonding the filament by the processing of heat embossing, for example.

For the method for bonding the spunbonded nonwoven fabric and the meltblown nonwoven fabric by heat fusion, the method in which the whole of the contact surfaces of the spunbonded nonwoven fabric and the meltblown nonwoven fabric are bonded by heat and a method in which part of the contact surfaces of the spunbonded nonwoven fabric and the meltblown nonwoven fabric are bonded by heat may be used. In the present invention, a method in which part of the contact surfaces of the spunbonded nonwoven fabric and the meltblown nonwoven fabric are bonded by the heat embossing method is preferable. In this case, the bonded area (which is equivalent to the area impressed by the embossing roll) is 5 to 35%, preferably 10 to 30%, of the contact surface of the spunbonded nonwoven fabric and the meltblown nonwoven fabric. If the bonded area is within the aforesaid range, the soft nonwoven fabric laminate, having a good balance between peel strength (bonding strength) and softness, is excellent.

Examples of the hot melt adhesive used in the method in which the spunbonded nonwoven fabric and the meltblown nonwoven fabric are bonded by use of an adhesive include, for example, adhesives based on resins such as vinyl acetate and polyvinyl alcohol and adhesives based on rubbers such as styrene-butadiene and styrene-isoprene. Further, examples of the solvent-based adhesive used in the present invention include, for example, adhesives which are based on rubbers such as styrene-butadiene, styrene-isoprene and urethane, and organic solvents and water-based emulsions which are based on resins such as vinyl acetate and vinyl chloride. Out of these adhesives, hot melt adhesives based on rubbers such as styrene-isoprene and styrene-butadiene are preferable in that they do not impair the characteristic hand of the spunbonded nonwoven fabric.

The soft nonwoven fabric laminate of the present invention obtained by the method as described above has satisfactory uniformity and excellent gas permeability, water impermeability and softness. In addition, this soft nonwoven fabric laminate also has excellent napping resistance because the surface of one or both sides of the laminate is formed of the spunbonded nonwoven fabric.

The KOSHI value, an index of softness, of the soft nonwoven fabric laminate of the present invention is normally less than 10, preferably not more than 9, and the interlaminar peel strength between the spunbonded nonwoven fabric and the meltblown nonwoven fabric is normally not less than 30 g/5 cm, preferably not less than 40 g/5 cm. Further, the water impermeability is normally not less than 150 mm Aq, preferably not less than 200 mm Aq.

The soft nonwoven fabric laminate of the present invention may be applied to a wide variety of sanitary goods, household materials, industrial materials and medical materials. Especially of its excellent softness, gas permeability and water impermeability, the soft nonwoven fabric laminate of the present invention is used suitably for a material for sanitary and packaging materials and applied to substrates of disposable diapers, sanitary napkins, poultice materials, etc. and raw materials for bed covers, etc. as a material for sanitary goods, and to compact disc bags, food packaging materials, clothing covers, etc. as a material for packaging materials.

### EXAMPLES

In the following Experiment Examples is given a specific explanation of the present invention. However, it is to be understood that the present invention is not be limited in any way by these Examples and Comparative Examples.

Further, the determination of the KOSHI value, peel strength, MFR and water impermeability in the Examples was made by the following methods:

### (1) Determination of the KOSHI Value

The tensile, shear strength, compression, surface friction and bending tests were conducted by use the KES-FB system available from Kato Tech Co., Ltd. using knit high-sensitivity conditions for the test conditions. The KOSHI value (as the value decreases, softness improves) was determined by carrying out a calculation by a formula using the results of the tests as parameters under knit underwear (summer) conditions. (As this value falls, softness improves.)

### (2) Determination of Peel Strength

Test specimens 5 cm in width and 20 cm in length were cut from the nonwoven laminate obtained. Peel strength was measured by peeling the layers of the laminate at the end of the test specimen, getting the end of each peeled layer gripped in the chucks of a tensile strength testing machine, and peeling the layers of the test specimen. As the testing conditions, the distance between the chucks was set at 20 mm, and the rate of pulling was set at 100 mm/minute. The average of the measurements of 5 test specimens was taken as peel strength (which means the same thing as bonding strength as used in this application).

### (3) Determination of MFR

The MFR of a propylene homopolymer and a propylene-ethylene random copolymer was measured at a load of 2.16 kg and a temperature of 230°C in accordance with ASTM D1238. The MFR of an ethylene homopolymer was measured at a load of 2.16 kg and a temperature of 190°C in accordance with ASTM D1238.

### (4) Determination of Water Impermeability

Water impermeability was determined in accordance with JIS 1096L

### Example 1:

A concentric core-sheath-type conjugate fiber having the core made of a propylene-ethylene random copolymer and the sheath made of an ethylene homopolymer (the ratio by weight between core and sheath was 20:80) was melt-spun by using the propylene-ethylene random copolymer having the ethylene content of 0.5 mol%, the Mw/Mn of 3.0 and the MFR of 65 g/10 minutes and the ethylene homopolymer having the density of 0.948 g/cm³, the Mw/Mn of 3.0 and the MFR of 30 g/10 minutes, and a spunbonded nonwoven fabric (S-1) having the basis weight of 23 g/m² and the component fiber having a fineness of 2 d(denier) was produced by depositing the conjugate fiber on the collection surface.

On the other hand, a meltblown nonwoven fabric (M-1) which comprised a fiber having an average diameter of 3 µm and had the basis weight of 6 g/m² was produced by the meltblown method in which a composition comprising 98.8 wt% of a propylene homopolymer having the MFR of 900 g/10 minutes and 1.2 wt% of an ethylene-α-olefin copolymer (available from Mitsui Chemicals, Inc.; registered trademark: TAFMER A) was melted by use of an extruder and the melted material was blown from a spinneret having apertures having a diameter of 0.38 mm, while at the same time hot air was blown at the outlets of the apertures.

Next, the aforesaid spunbonded nonwoven fabric (S-1) and the meltblown nonwoven fabric (M-1) were put on top of each other, and these two layers of nonwoven fabrics were combined into one at a linear pressure of 60 kg/cm by use of a heat embossing roll heated to 120°C which had an impression area ratio (ratio of the impression area to the total area) of 18%. As a result, a nonwoven fabric laminate was obtained. The KOSHI value, peel strength and water impermeability of the nonwoven fabric laminate thus obtained were measured. Results of the measurement are shown in Table 1.

### Example 2:

A meltblown nonwoven fabric (M-2) having a basis weight of 6 g/m² which comprises a fiber having an average fiber diameter of 3 µm was produced by the same method as described in Example 1 except that a composition comprising 75 wt% of a propylene homopolymer having the MFR of 900 g/10 minutes and 25 wt% of an ethylene-α-olefin copolymer (available from Mitsui chemicals, Inc.; registered trademark: TAFMER A) was used for the raw material resin for the meltblown nonwoven fabric.

Next, the spunbonded nonwoven fabric (S-1) produced as described in Example 1 and the aforesaid meltblown nonwoven fabric (M-2) were put on top of each other, and these two layers of nonwoven fabrics were combined into one by use of a heat embossing roll in the same manner as described in Example 1. As a result, a nonwoven fabric laminate was obtained. The measurement results of the nonwoven fabric laminate thus obtained are shown in Table 1.

### Comparative Example 1:

A meltblown nonwoven fabric (M-3) having a basis weight of 6 g/m² which comprises a fiber having an average fiber diameter of 3 µm was produced by the same method as described in Example 1 except that a propylene homopolymer having the MFR of 900 g/10 minutes alone was used for the raw material resin for the meltblown nonwoven fabric.

Next, the spunbonded nonwoven fabric (S-1) produced as described in Example 1 and the aforesaid meltblown nonwoven fabric (M-3) were put on top of each other, and these two layers of nonwoven fabrics were combined into one by use of a heat embossing roll in the same manner as described in Example 1. As a result, a nonwoven fabric laminate was obtained. The measurement results of the nonwoven fabric laminate thus obtained are shown in Table 1.

### Comparative Example 2:

A meltblown nonwoven fabric (M-4) was produced by the same method as described in Example 1 except that a composition comprising 45 wt% of a propylene homopolymer having the MFR of 900 g/10 minutes and 55 wt% of an ethylene-α-olefin copolymer (available from Mitsui Chemicals, Inc.; registered trademark: TAFMER A) was used for the raw material resin for the meltblown nonwoven fabric. The average fiber diameter of the resulting meltblown nonwoven fabric was larger average at 3 to 4 µm, and there was an occurrence of resin shots.

Next, the spunbonded nonwoven fabric (S-1) produced as described in Example 1 and the aforesaid meltblown nonwoven fabric (M-4) were put on top of each other, and a nonwoven fabric laminate was obtained by combining these two layers into one by use of the heat embossing roll in the same manner as described in Example 1. The measurement results of the nonwoven fabric laminate thus obtained are shown in Table 1.

### Example 3:

A concentric core-sheath-type conjugate fiber having the core made of a propylene homopolymer and the sheath made of an ethylene homopolymer (the ratio by weight between core and sheath was 20:80) was melt-spun by using the propylene homopolymer having the Mw/Mn of 3.0 and the MFR of 65 g/10 minutes and the ethylene homopolymer having the density of 0.948 g/cm³, the Mw/Mn of 3.0 and the MFR of 20 g/10 minutes, and a spunbonded nonwoven fabric (S-2) having the basis weight of 23 g/m² and the component fiber having a fineness of 2 d was produced by depositing the conjugate fiber on the collection surface.

Next, the aforesaid spunbonded nonwoven fabric (S-2) and the meltblown nonwoven fabric (M-2) produced as described in Example 2 were put on top of each other, and these two layers of nonwoven fabrics were combined into one by use of a heat embossing roll in the same manner as described in Example 1. As a result, a nonwoven fabric laminate was obtained. The measurement results of the nonwoven fabric laminate thus obtained are shown in Table 1.

### Example 4:

A spunbonded nonwoven fabric (S-3) having the basis weight of 23 g/m² and the component fiber having a fineness of 2 d was produced by the same method as described in Example 3 except that a propylene-ethylene random copolymer having the ethylene content of 0.5 mol%, the Mw/Mn of 3.0 and the MFR of 65 g/10 minutes was used for the propylene homopolymer.

Next, the aforesaid spunbonded nonwoven fabric (S-3) and the meltblown nonwoven fabric (M-2) produced as described in Example 2 were put on top of each other, and a nonwoven fabric laminate was obtained by combining these two layers into one by use of a heat embossing roll in the same manner as described in Example 1. The measurement results of the nonwoven fabric laminate thus obtained are shown in Table 1.

### Example 5:

A spunbonded nonwoven fabric (S-4) having the basis weight of 23 g/m² and the component fiber having a fineness of 2 d was produced by the same method as described in Example 3 except that a propylene-ethylene random copolymer having the ethylene content of 4.0 mol%, the Mw/Mn of 3.0 and the MFR of 65 g/10 minutes was used for the propylene homopolymer.

Next, the aforesaid spunbonded nonwoven fabric (S-4) and the meltblown nonwoven fabric (M-2) produced as described in Example 2 were put on top of each other, and a nonwoven fabric laminate was obtained by combining these two layers into one by use of a heat embossing roll in the same manner as described in Example 1. The measurement results of the nonwoven fabric laminate thus obtained are shown in Table 1.

### Comparative Example 3:

An attempt was made to produce a spunbonded nonwoven fabric by the same method as described in Example 3 except that a propylene-ethylene random copolymer having the ethylene content of 5.5 mol%, the Mw/Mn of 3.0 and the MFR of 65 g/10 minutes was used for the propylene homopolymer, but the attempt failed due to poor spinnability.

### Example 6:

A concentric core-sheath-type conjugate fiber having the core made of a propylene-ethylene random copolymer and the sheath made of an ethylene homopolymer (the ratio by weight between core and sheath was 5:95) was melt-spun by using the propylene-ethylene random copolymer having the ethylene content of 0.5 mol%, the Mw/Mn of 3.0 and the MFR of 65 g/10 minutes and the ethylene homopolymer having the density of 0.948 g/cm³, the Mw/Mn of 3.0 and the MFR of 20 g/10 minutes, and a spunbonded nonwoven fabric (S-5) having the basis weight of 23 g/m² and the component fiber having a fineness of 2 d was produced by depositing the conjugate fiber on the collection surface.

Next, the aforesaid spunbonded nonwoven fabric (S-5) and the meltblown nonwoven fabric (M-2) produced as described in Example 2 were put on top of each other, and these two layers of nonwoven fabrics were combined into one by use of a heat embossing roll in the same manner as described in Example 1. As a result, a nonwoven fabric laminate was obtained. The measurement results of the nonwoven fabric laminate thus obtained are shown in Table 1.

### Comparative Example 4:

An attempt was made to produce a spunbonded nonwoven fabric by the same method as described in Example 6 except that the ratio by weight of the core and the sheath was set at 3:97. However, the attempt failed due to poor spinnability.

### Comparative Example 5:

A spunbonded nonwoven fabric (S-6) having the basis weight of 23 g/m² and the component fiber having a fineness of 2 d was produced in the same manner as described in Example 6 except that the ratio by weight of the core and the sheath was set at 70:30.

Next, the aforesaid spunbonded nonwoven fabric (S-6) and the meltblown nonwoven fabric (M-2) produced as described in Example 2 were put on top of each other, and a nonwoven fabric laminate was obtained by combining these two layers into one by use of a heat embossing roll in the same manner as described in Example 1. The measurement results of the nonwoven fabric laminate thus obtained are shown in Table 1.

### Comparative Example 6:

A spunbonded nonwoven fabric (S-7) having the basis weight of 23 g/m² and the component fiber having a fineness of 2 d was produced by depositing a fiber obtained by melt-spinning only a propylene-ethylene random copolymer having the ethylene content of 0.5 mol%, the Mw/Mn of 3.0 and the MFR of 65 g/10 minutes on the collection surface.

Next, the aforesaid spunbonded nonwoven fabric (S-7) and the meltblown nonwoven fabric (M-2) produced as described in Example 2 were put on top of each other, and a nonwoven fabric laminate was obtained by combining these two layers into one by use of a heat embossing roll in the same manner as described in Example 1. The measurement results of the nonwoven fabric laminate thus obtained are shown in Table 1.

### Example 7:

A concentric core-sheath-type conjugate fiber having the core made of a propylene-ethylene random copolymer and the sheath made of an ethylene homopolymer (the ratio by weight between core and sheath was 20:80) was melt-spun by using the propylene-ethylene random copolymer having the ethylene content of 0.5 mol%, the Mw/Mn of 3.0 and the MFR of 65 g/10 minutes and the ethylene homopolymer having the density of 0.917 g/cm³, the Mw/Mn of 3.0 and the MFR of 20 g/10 minutes, and a spunbonded nonwoven fabric (S-8) having the basis weight of 23 g/m² and the component fiber having a fineness of 2 d was produced by depositing the conjugate fiber on the collection surface.

Next, the aforesaid spunbonded nonwoven fabric (S-8) and the meltblown nonwoven fabric (M-2) produced as described in Example 2 were put on top of each other, and these two layers of nonwoven fabrics were combined into one by use of a heat embossing roll in the same manner as described in Example 1. As a result, a nonwoven fabric laminate was obtained. The measurement results of the nonwoven fabric laminate thus obtained are shown in Table 2.

### Comparative Example 7:

A spunbonded nonwoven fabric (S-9) having the basis weight of 23 g/m² and the component fiber having a fineness of 3.2 d was produced by the same method as described in Example 7 except that an ethylene homopolymer having the density of 0.870 g/cm³, the Mw/Mn of 3.0 and the MFR of 20 g/10 minutes for the ethylene-based polymer (b).

Next, the aforesaid spunbonded nonwoven fabric (S-9) and the meltblown nonwoven fabric (M-2) produced as described in Example 2 were put on top of each other, and these two layers of nonwoven fabrics were combined into one by use of a heat embossing roll in the same manner as described in Example 1. As a result, a nonwoven fabric laminate was obtained. The measurement results of the nonwoven fabric laminate thus obtained are shown in Table 2.

### Comparative Example 8:

An attempt was made to produce a spunbonded nonwoven fabric by the same method as described in Example 7 except that an ethylene-based homopolymer having the density of 0.980 g/cm³, the Mw/Mn of 3.0 and the MFR of 20 g/10 minutes was used for the ethylene-based polymer (b). However, the attempt failed due to poor spinnability.

### Example 8:

A concentric core-sheath-type conjugate fiber having the core made of a propylene-ethylene random copolymer and the sheath made of an ethylene homopolymer (the ratio by weight between core and sheath was 10:90) was melt-spun by using the propylene-ethylene random copolymer having the ethylene content of 0.5 mol%, the Mw/Mn of 2.0 and the MFR of 65 g/10 minutes and the ethylene homopolymer having the density of 0.948 g/cm³, the Mw/Mn of 3.0 and the MFR of 30 g/10 minutes, and a spunbonded nonwoven fabric (S-10) having the basis weight of 23 g/m² and the component fiber having a fineness of 2 d was produced by depositing the conjugate fiber on the collection surface.

Next, the aforesaid spunbonded nonwoven fabric (S-10) and the meltblown nonwoven fabric (M-2) produced as described in Example 2 were put on top of each other, and these two layers of nonwoven fabrics were combined into one by use of a heat embossing roll in the same manner as described in Example 1. As a result, a nonwoven fabric laminate was obtained. The measurement results of the nonwoven fabric laminate thus obtained are shown in Table 2.

### Example 9:

A spunbonded nonwoven fabric (S-11) having the basis weight of 23 g/m² and the component fiber having a fineness of 2 d was produced by the same method as described in Example 8 except that a propylene-ethylene random copolymer having the ethylene content of 0.5 mol%, the Mw/Mn of 3.0 and the MFR of 65 g/10 minutes was used for the propylene-based polymer (a).

Next, the aforesaid spunbonded nonwoven fabric (S-11) and the meltblown nonwoven fabric (M-2) produced as described in Example 2 were put on top of each other, and a nonwoven fabric laminate was obtained by combining these two layers into one by use of a heat embossing roll in the same manner as described in Example 1. As a result, a nonwoven fabric laminate was obtained. The measurement results of the nonwoven fabric laminate thus obtained are shown in Table 2.

### Comparative Example 9:

An attempt was made to produce a spunbonded nonwoven fabric by the same method as described in Example 8 except that a propylene-ethylene random copolymer having the ethylene content of 0.5 mol%, the Mw/Mn of 6.0 and the MFR of 65 g/10 minutes was used for the propylene-based polymer (a). However, the attempt failed due to poor spinnability.

### Example 10:

A concentric core-sheath-type conjugate fiber having the core made of a propylene-ethylene random copolymer and the sheath made of an ethylene homopolymer (the ratio by weight between core and sheath was 20:80) was melt-spun by using the propylene-ethylene random copolymer having the ethylene content of 0.5 mol%, the Mw/Mn of 2.0 and the MFR of 65 g/10 minutes and the ethylene homopolymer having the density of 0.948 g/cm³, the Mw/Mn of 1.5 and the MFR of 30 g/10 minutes, and a spunbonded nonwoven fabric (S-12) having the basis weight of 23 g/m² and the component fiber having a fineness of 2 d was produced by depositing the conjugate fiber on the collection surface.

Next, the aforesaid spunbonded nonwoven fabric (S-12) and the meltblown nonwoven fabric (M-2) produced as described in Example 2 were put on top of each other, and these two layers of nonwoven fabrics were combined into one by use of a heat embossing roll in the same manner as described in Example 1. As a result, a nonwoven fabric laminate was obtained. The measurement results of the nonwoven fabric laminate thus obtained are shown in Table 2.

### Example 11:

A spunbonded nonwoven fabric (S-13) having the basis weight of 23 g/m² and the component fiber having a fineness of 2 d was produced by the same method as described in Example 10 except that an ethylene homopolymer having the density of 0.948 g/cm³, the Mw/Mn of 2.5 and the MFR of 30 g/10 minutes was used for the ethylene-based polymer (b).

Next, the aforesaid spunbonded nonwoven fabric (S-13) and the meltblown nonwoven fabric (M-2) produced as described in Example 2 were put on top of each other, and a nonwoven fabric laminate was obtained by combining these two layers into one by use of a heat embossing roll in the same manner as described in Example 1. The measurement results of the nonwoven fabric laminate thus obtained are shown in Table 2.

### Comparative Example 10:

An attempt was made to produce a spunbonded nonwoven fabric by the same method as described in Example 10 except that an ethylene homopolymer having the density of 0.948 g/cm³, the Mw/Mn of 6.0 and the MFR of 30 g/10 minutes was used for the ethylene-based polymer (b). However, the attempt failed due to poor spinnability.

**Table 1**

| | Spunbonded nonwoven fabric | | | | | | | | Meltblown nonwoven fabric | Nonwoven fabric laminate | | |
|---|---|---|---|---|---|---|---|---|---|---|---|---|
| | Propylene-based polymer (a) | | | Ethylene-based polymer (b) | | | (a)/(b) | Fineness | | | | |
| | MFR (g/ 10min) | Mw/Mn | Ethylene content (mol%) | MFR (g/ 10min) | Mw/Mn | Density (g/cm') | ratio by wt. | (d) | Polyolefin-based elastomer (c) content (wt%) | KOSHI value | Peel strength (g/5cm) | Water impermeability (mm Aq) |
| Comp. Example 1 | 65 | 3.0 | 0.5 | 30 | 3.0 | 0.948 | 20/80 | 2.0 | 0 | 8.87 | Delamination | 300 |
| Example 1 | 65 | 3.0 | 0.5 | 30 | 3.0 | 0.948 | 20/80 | 2.0 | 1.2 | 8.60 | 53 | 250 |
| Example 2 | 65 | 3.0 | 0.5 | 30 | 3.0 | 0.948 | 20/80 | 2.0 | 25 | 8.40 | 55 | 200 |
| Comp. Example 2 | 65 | 3.0 | 0.5 | 30 | 3.0 | 0.948 | 20/80 | 2.0 | 55 | 8.41 | 70 | 100 |
| Example 3 | 65 | 3.0 | 0 | 20 | 3.0 | 0.948 | 20/80 | 2.0 | 25 | 9.50 | 44 | 200 |
| Example 4 | 65 | 3.0 | 0.5 | 20 | 3.0 | 0.948 | 20/80 | 2.0 | 25 | 8.60 | 49 | 210 |
| Example 5 | 65 | 3.0 | 4.0 | 20 | 3.0 | 0.948 | 20/80 | 2.0 | 25 | 8.70 | 48 | 203 |
| Comp. Example 3 | 65 | 3.0 | 5.5 | 20 | 3.0 | 0.948 | 20/80 | *1 | - | - | - | - |
| Comp. Example 4 | 65 | 3.0 | 0.5 | 20 | 3.0 | 0.948 | 3/97 | *1 | - | - | - | - |
| Example 6 | 65 | 3.0 | 0.5 | 20 | 3.0 | 0.948 | 5/95 | 2.0 | 25 | 8,40 | 60 | 220 |
| Example 4 | 65 | 3.0 | 0.5 | 20 | 3.0 | 0.948 | 20/80 | 2.0 | 25 | 8.60 | 49 | 210 |
| Comp. Example 5 | 65 | 3.0 | 0.5 | 20 | 3.0 | 0.948 | 70/30 | 2.0 | 25 | 10.5 | 45 | 210 |
| Comp. Example 6 | 65 | 3.0 | 0.5 | - | - | - | 100/0 | 2.0 | 25 | 11 | Peeling impossible | 200 |

| | | | | | | | | | | | | |
|---|---|---|---|---|---|---|---|---|---|---|---|---|
| *1: Poor spinnability | | | | | | | | | | | | |

**Table 2**

| | Spunbonded nonwoven fabric | | | | | | | | Meltblown nonwoven fabric | Nonwoven fabric laminate | | |
|---|---|---|---|---|---|---|---|---|---|---|---|---|
| | Propylene-based polymer (a) | | | Ethylene-based polymer (b) | | | (a)/(b) | Fineness | | | | |
| | MFR (g/ 10min) | Mw/Mn | Ethylene content (mol%) | MFR (g/ 10min) | Hw/Mn | Density (g/cm³) | ratio by wt. | (d) | Polyolefin-based elastomer (c) content (wt%) | KOSHI value | Peel strength (g/5cm) | Water impermeability (mm Aq) |
| Comp. Example 7 | 65 | 3.0 | 0.5 | 20 | 3.0 | 0.870 | 20/80 | 3.2 | 25 | 10.3 | 55 | 200 |
| Example 7 | 65 | 3.0 | 0.5 | 20 | 3.0 | 0.917 | 20/80 | 2.0 | 25 | 8.80 | 40 | 215 |
| Example 4 | 65 | 3.0 | 0.5 | 20 | 3.0 | 0.948 | 20/80 | 2.0 | 25 | 8.60 | 49 | 210 |
| Comp. Example 8 | 65 | 3.0 | 0.5 | 20 | 3.0 | 0.980 | 20/80 | *1 | - | - | - | - |
| Example 8 | 65 | 2.0 | 0.5 | 30 | 3.0 | 0.948 | 10/90 | 2.0 | 25 | 8.87 | 39 | 205 |
| Example 9 | 65 | 3.0 | 0.5 | 30 | 3.0 | 0.948 | 10/90 | 2.0 | 25 | 8.55 | 40 | 203 |
| Comp. Example 9 | 65 | 6.0 | 0.5 | 30 | 3.0 | 0.948 | 10/90 | *1 | - | - | - | - |
| Example 10 | 65 | 2.0 | 0.5 | 30 | 1.5 | 0.948 | 20/80 | 2.0 | 25 | 8.90 | 55 | 205 |
| Example 11 | 65 | 2.0 | 0.5 | 30 | 2.5 | 0.948 | 20/80 | 2.0 | 25 | 8.80 | 40 | 206 |
| Comp. Example 10 | 65 | 2.0 | 0.5 | 30 | 6.0 | 0.948 | 20/80 | *1 | - | - | - | - |

| | | | | | | | | | | | | |
|---|---|---|---|---|---|---|---|---|---|---|---|---|
| *1: Poor spinnability | | | | | | | | | | | | |

### POSSIBILITY OF INDUSTRIAL USE

The soft nonwoven fabric laminate of the present invention has satisfactory uniformity and excellent softness, gas permeability and water impermeability. In addition, it shows excellent adhesion between the spunbonded nonwoven fabric layer and the meltblown nonwoven fabric layer. Because of this, the soft nonwoven fabric laminate of the present invention may be used suitably for a broad range of applications for which nonwoven fabrics have been used traditionally. It may be used suitably especially for applications including raw materials for sanitary goods such as disposable diapers and sanitary napkins, substrates such as poultices and packaging materials.

## Claims

1. A soft nonwoven fabric laminate which comprises at least one spunbonded nonwoven fabric layer and at least one meltblown nonwoven fabric layer and one surface or both surfaces of which are a spunbonded nonwoven fabric layer; with the aforesaid spunbonded nonwoven fabric comprising a conjugate fiber:
which comprises:
(A) A propylene-based polymer (a) having the physical properties as described in (1) and (2) below:
(1) The ethylene content is 0 to 5 mol%;
(2) Mw/Mn is 2 to 4;
(B) An ethylene-based polymer (b) having the physical properties as described in (3) and (4) below and forming at least part of the fiber surface:
(3) The density is 0.880 to 0.970 g/cm³;
(4) Mw/Mn is 1.5 to 4; and
in which the ratio by weight between (a) and (b) ((a)/(b)) is 5/95 to 50/50; and
with the aforesaid meltblown nonwoven fabric comprising a polyolefin composition which comprises a polyolefin-based elastomer (c) and a propylene-based polymer (d) and contains the polyolefin-based elastomer (c) at a ratio of not less than one wt% but not more than 50 wt%.

2. A soft nonwoven fabric laminate as claimed in claim 1, wherein the conjugate fiber composing the aforesaid spunbonded nonwoven fabric is a concentric core-sheath-type conjugate fiber making up the core comprising a propylene-based polymer (a) and the sheath comprising an ethylene-based polymer (b).

3. A soft nonwoven fabric laminate as claimed in claim 1, wherein the conjugate fiber composing the aforesaid spunbonded nonwoven fabric is an eccentric core-sheath-type conjugate fiber comprising the core made up of the propylene-based polymer (a) and the sheath made up of the ethylene-based polymer (b).

4. A soft nonwoven fabric laminate as claimed in claim 1, wherein the conjugate fiber comprising the aforesaid spunbonded nonwoven fabric is a side-by-side-type conjugate fiber which comprises the propylene-based polymer (a) and the ethylene-based polymer (b).

5. A soft nonwoven fabric laminate as claimed in claims 1 through 4, wherein the aforesaid spunbonded nonwoven fabric layer and the aforesaid meltblown nonwoven fabric layer are thermally bonded by the processing of heat embossing.

6. A soft nonwoven fabric laminate as claimed in claim 5, wherein the bonded area of the spunbonded nonwoven fabric layer and the meltblown nonwoven fabric layer is 5% to 35% of the area of contact between the aforesaid spunbonded nonwoven fabric layer and the aforesaid meltblown nonwoven fabric layer.

7. A soft nonwoven fabric laminate as claimed in any of claims 1 through 6, wherein the KOSHI value is less than 10.

8. A soft nonwoven fabric laminate as claimed in any of claims 1 through 7, wherein the peel strength between the aforesaid spunbonded nonwoven fabric layer and the aforesaid meltblown nonwoven fabric layer is not less than 30 g/5 cm.

9. A material for use in sanitary goods which comprises the soft nonwoven fabric laminate as claimed in claims 1 through 8.

10. A material for use in packaging materials which comprises the soft nonwoven fabric laminate as claimed in claims 1 through 8.

## Patentansprüche

1. Weiches Vliesstofflaminat, das zumindest eine spunbonded Vliesstoffschicht und zumindest eine meltblown Vliesstoffschicht umfaßt, wovon eine Oberfläche oder beide Oberflächen eine spunbonded Vliesstoffschicht ist/sind; worin der vorstehende spunbonded Vliesstoff eine Konjugatfaser umfaßt, welche umfaßt:
(A) ein Polymer auf Propylenbasis (a), das die physikalischen Eigenschaften aufweist, wie sie nachstehend in (1) und (2) beschrieben sind:
(1) der Ethylengehalt beträgt 0 bis 5 mol%;
(2) Mw/Mn beträgt 2 bis 4;
(B) ein Polymer auf Ethylenbasis (b), das die physikalischen Eigenschaften aufweist, wie sie nachstehend in (3) und (4) beschrieben sind, und das zumindest einen Teil der Faseroberfläche bildet:
(3) die Dichte beträgt 0,880 bis 0,970 g/cm³;
(4) Mw/Mn beträgt 1,5 bis 4; und
worin das Gewichtsverhältnis zwischen (a) und (b) ((a)/(b)) 5/95 bis 50/50 beträgt; und
worin der vorstehend erwähnte meltblown Vliesstoff eine Polyolefinzusammensetzung umfaßt, die ein Elastomer auf Polyolefinbasis (c) und ein Polymer auf Propylenbasis (d) umfaßt, und das Elastomer auf Polyolefinbasis (c) in einem Anteil von nicht weniger als 1 Gew.%, jedoch nicht mehr als 50 Gew.%., enthält.

2. Weiches Vliesstofflaminat gemäß Anspruch 1, worin die Konjugatfaser, die den vorstehenden spunbonded Vliesstoff ausmacht, eine Konjugatfaser vom konzentrischen Kern-Hülle-Typ ist, die den Kern, der ein Polymer auf Propylenbasis (a) umfaßt, und die Hülle, die ein Polymer auf Ethylenbasis (b) umfaßt, bildet.

3. Weiches Vliesstofflaminat gemäß Anspruch 1, worin die Konjugatfaser, die den vorstehenden spunbonded Vliesstoff ausmacht, eine Konjugatfaser vom exzentrischen Kern-Hülle-Typ ist, die den Kern, der aus dem Polymer auf Propylenbasis (a) hergestellt ist, und die Hülle, die aus dem Polymer auf Ethylenbasis (b) hergestellt ist, umfaßt.

4. Weiches Vliesstofflaminat gemäß Anspruch 1, worin die Konjugatfaser, die den vorstehenden spunbonded Vliesstoff ausmacht, eine Konjugatfaser vom Seite-an-Seite-Typ ist, die das Polymer auf Propylenbasis (a) und das Polymer auf Ethylenbasis (b) umfaßt.

5. Weiches Vliesstofflaminat gemäß den Ansprüchen 1 bis 4, worin die vorstehend erwähnte spunbonded Vliesstoffschicht und die vorstehend erwähnte meltblown Vliesstoffschicht durch Verarbeiten mit Wärmeprägen thermisch verbunden sind.

6. Weiches Vliesstofflaminat gemäß Anspruch 5, worin die Bindungsfläche der spunbonded Vliesstoffschicht und der meltblown Vliesstoffschicht 5 bis 35 % der Kontaktfläche zwischen der vorstehenden spunbonded Vliesstoffschicht und der vorstehenden meltblown Vliesstoffschicht ausmacht.

7. Weiches Vliesstofflaminat gemäß irgendeinem der Ansprüche 1 bis 6, worin der KOSHI-Wert weniger als 10 beträgt.

8. Weiches Vliesstofflaminat gemäß irgendeinem der Ansprüche 1 bis 7, worin die Ablösefestigkeit zwischen der vorstehenden spunbonded Vliesstoffschicht und der vorstehenden meltblown Vliesstoffschicht nicht weniger als 30 g/5 cm beträgt.

9. Material zur Verwendung in Hygiene/Sanitärartikeln, welches das weiche Vliesstofflaminat gemäß den Ansprüchen 1 bis 8 umfaßt.

10. Material zur Verwendung in Verpackungsmaterialien, welches das weiche Vliesstofflaminat gemäß den Ansprüchen 1 bis 8 umfaßt.

## Revendications

1. Stratifié de tissu non-tissé flexible qui comprend au moins une couche de tissu non-tissé filé-lié et au moins une couche de tissu non-tissé soufflé à l'état fondu dont une surface ou les deux surfaces sont une couche de tissu non-tissé filé-lié avec le tissu non-tissé filé-lié précité comprenant une fibre conjuguée :
qui comprend :
(A) un polymère à base de propylène (a) ayant les propriétés physiques décrites dans (1) et (2) ci-après :
(1) la teneur d'éthylène est de 0 à 5 % en moles ;
(2) le poids moléculaire en poids/poids moléculaire en nombre (Mw/Mn) est de 2 à 4 ;
(B) un polymère à base d'éthylène (b) ayant les propriétés physiques comme décrit dans (3) et (4) ci-après et formant au moins une partie de la surface de fibre :
(3) la densité est de 0,880 à 0,970 g/cm³ ;
(4) Mw/Mn est de 1,5 à 4 ; et
dans lequel le rapport en poids entre (a) et (b) ((a) / ((b) est de 5/95 à 50/50 ; et
le tissu non-tissé soufflé à l'état fondu précité comprend une composition de polyoléfine qui comporte un élastomère à base de polyoléfine (c) et un polymère à base de propylène (d) et renferme l'élastomère à base de polyoléfine (c) à un rapport qui n'est pas inférieur à 1 % en poids mais pas supérieur à 50 % en poids.

2. Stratifié de tissu non-tissé flexible selon la revendication 1, dans lequel la fibre conjuguée constituant le tissu non-tissé filé-lié précité est une fibre conjuguée du type âme-gaine concentrique avec l'âme comprenant un polymère à base de propylène (a) et la gaine comprenant un polymère à base d'éthylène (b).

3. Stratifié de tissu non-tissé flexible selon la revendication 1, dans lequel la fibre conjuguée constituant le tissu non-tissé filé-lié précité est une fibre conjuguée du type âme-gaine excentrique comprenant l'âme constituée du polymère à base de propylène (a) et la gaine constituée du polymère à base d'éthylène (b).

4. Stratifié de tissu non-tissé flexible selon la revendication 1, dans lequel la fibre conjuguée comprenant le tissu non-tissé filé-lié précité est une fibre conjuguée de type côte à côte qui comprend le polymère à base de propylène (a) et le polymère à base d'éthylène (b).

5. Stratifié de tissu non-tissé flexible selon les revendications 1 à 4, dans lequel la couche de tissu non-tissé filé-lié précitée et la couche de tissu non-tissé soufflé à l'état fondu précitée sont liées thermiquement par le procédé de gaufrage à la chaleur.

6. Stratifié de tissu non-tissé flexible selon la revendication 5, dans lequel l'étendue liée de la couche de tissu non-tissé filé-lié et la couche de tissu non-tissé soufflée à l'état fondu représente 5 % à 35 % de l'étendue de contact entre la couche de tissu non-tissé filé-lié précitée et la couche de tissu non-tissé soufflé à l'état fondu précitée.

7. Stratifié de tissu non-tissé flexible selon l'une quelconque des revendications 1 à 6, dans lequel la valeur KOSHI est inférieure à 10.

8. Stratifié de tissu non-tissé flexible selon l'une quelconque des revendications 1 à 7, dans lequel la résistance à l'arrachement entre la couche de tissu non-tissé filé-lié précitée et la couche de tissu non-tissé soufflé à l'état fondu précitée n'est pas inférieure à 30 g/5cm.

9. Matériau destiné à être utilisé dans les articles hygiéniques qui comprend le stratifié de tissu non-tissé flexible selon l'une quelconque des revendications 1 à 8.

10. Matériau destiné à être utilisé dans les matériaux d'emballage qui comprend le stratifié de tissu non-tissé flexible selon l'une quelconque des revendications 1 à 8.
